# EUROPEAN PATENT APPLICATION

(11) **EP 4 806 882 A1**
(43) Date of publication of application: **16.09.2026**
(21) Application number: 24890358.5
(22) Date of filing: 29.09.2024
(51) Int. Cl.: C07C 231/12, C07C 233/18

(54) **PREPARATION METHOD FOR N-ALKENYL AMIDE**

(30) Priority: 17.11.2023 CN 202311542418
(71) Applicant: China Petroleum & Chemical Corporation, Beijing 100728 (CN); Sinopec (Beijing) Research Institute of Chemical Industry Co., Ltd., Beijing 100013 (CN)
(72) Inventor: FAN, Rong, Beijing 100013 (CN); YI, Zhuo, Beijing 100013 (CN); LIU, Xi, Beijing 100013 (CN); HU, Xiaona, Beijing 100013 (CN); LI, Yajing, Beijing 100013 (CN); ZHAO, Ruotong, Beijing 100013 (CN); ZHANG, Ruiqi, Beijing 100013 (CN); YANG, Jinbiao, Beijing 100013 (CN); SHANG, Dansen, Beijing 100013 (CN)
(74) Representative: Hoffmann Eitle
(86) International application number: PCT/CN2024/122281
(87) International publication number: WO 2025/103000

(57) **Abstract**

A preparation method for a N-alkenyl amide. The method comprises: under an esterification reaction condition and in the presence of an esterification reaction catalyst, contacting a compound represented by formula (1) with a compound represented by formula (2-1) to enable a first reaction to occur, and then subjecting the obtained first reaction product to a cracking reaction. The method involves simple operation, a high product yield, excellent selectivity and simple product separation, and particularly, raw materials can be recycled, thus greatly reducing the production cost and allowing for an economic production route.

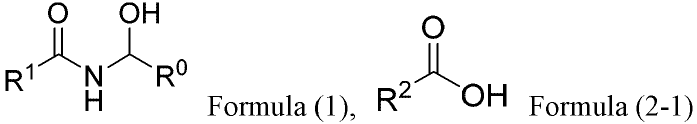

## Description

### CROSS-REFERENCE TO RELATED APPLICATIONS

The application claims the benefit of the China patent application No. "202311542418.0", filed on November 17, 2023, the content of which is specifically and entirely incorporated herein by reference.

### TECHNICAL FIELD

The present invention relates to the technical field of oilfield exploitation, in particular to a preparation method for a N-alkenyl amide.

### BACKGROUND ART

N-alkenyl amide is a new kind of functional monomer, which has attracted attentions due to its advantages such as excellent water solubility, high polymerization activity, non-toxicity and harmlessness. Its polymers are widely applied in oil extraction, papermaking, water treatment, and other fields. Most of the early manufacturing processes prepare the N-alkenyl formamide by using the high temperature cracking reaction of precursors. The monomer has poor thermal stability, thus the product has the low yield and selectivity.

In response to the above problems, Changchun Institute of Applied Chemistry in China discloses a method for preparing N-alkenyl amide by using formamide, acetaldehyde and anhydride in CN112047854A. The method can significantly reduce the temperature of cracking reaction, however, the process will produce a large amount of acidic substances, and the treatment of waste liquid is difficult and expensive, thus the method can hardly be industrially applied in practice.

### SUMMARY OF THE INVENTION

The present invention aims to overcome the defects in the prior art, and provides a preparation method for a N-alkenyl amide, the method involves simple operation, a high product yield, excellent selectivity and simple product separation process, and particularly, raw materials can be recycled, thereby greatly reducing the production cost.

In order to achieve the above object, the first aspect of the present invention provides a preparation method for a N-alkenyl amide, the method comprises: under an esterification reaction condition and in the presence of an esterification reaction catalyst, contacting a compound represented by formula (1) with a compound represented by formula (2-1) to carry out a first reaction, and then subjecting the obtained first reaction product to a cracking reaction; 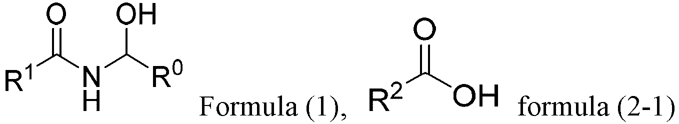
wherein R⁰ in formula (1) is C1-C5 alkyl group, R¹ is H or C1-C8 hydrocarbyl group;
R² in formula (2-1) is H or C1-C8 hydrocarbyl group.

The second aspect of the present invention provides a preparation method for a N-alkenyl amide, the method comprises: under basic conditions, subjecting an aldehyde and an amide to obtain a compound represented by formula (1) through the nucleophilic addition reaction, and producing a N-alkenyl amide with the compound represented by formula (1) according to the aforementioned method; 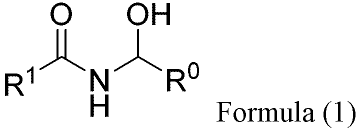 wherein R⁰ in formula (1) is C1-C5 alkyl group, R¹ is H or C1-C8 hydrocarbyl group.

Due to the above technical scheme, the present invention at least produces the favorable effects as follows:
(1) The preparation method for a N-alkenyl amide according to the present invention includes performing a reaction by using a carboxylic acid as one of raw materials, and the carboxylic acid is generated after a cracking reaction, so that the raw material carboxylic acid is not actually consumed, and the new impurity is not introduced, and the remaining material after extracting a target product N-alkenyl amide from a mixture obtained following the reaction can be directly recycled for the next reaction to prepare a N-alkenyl amide.
(2) The method of the present invention uses carboxylic acid as a raw material, and utilizes the carboxylate as a catalyst, the product has a high yield and selectivity.
(3) The present invention can produce the product with a low cost and a simple post-treatment under the circumstance of ensuring the high yield and selectivity, both the carboxylic acid and the carboxylate are recyclable, the emission amounts of three wastes (wastewater, exhaust gas, solid waste) are low, the production costs are greatly reduced, thus the method provided by the invention has the prospects for industrial applications.
(4) The preparation method of the present invention does not involve a high pressure process, and imposes a low requirement on the material of the reaction equipment.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The present invention provides a preparation method for a N-alkenyl amide, the method comprises: under an esterification reaction condition and in the presence of an esterification reaction catalyst, contacting a compound represented by formula (1) with a compound represented by formula (2-1) to carry out a first reaction, and then subjecting the obtained first reaction product to a cracking reaction; 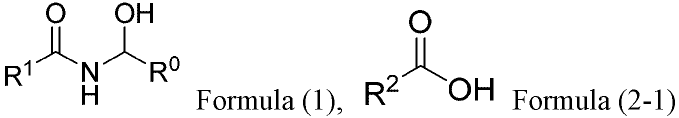
wherein R⁰ in formula (1) is C1-C5 alkyl group, R¹ is H or C1-C8 hydrocarbyl group;
R² in formula (2-1) is H or C1-C8 hydrocarbyl group.

The present inventors have discovered in researches that by using the above method, a carboxylic acid is used as a raw material for carrying out the reaction, the carboxylic acid will be generated after the cracking reaction, so that the raw material carboxylic acid is not actually consumed, and the new impurity is not introduced, and the remaining material after extracting a target product N-alkenyl amide from a mixture obtained following the reaction can be directly recycled for the next reaction to prepare a N-alkenyl amide. The reaction can be carried out under mild conditions (e.g., without involving high pressure), such that the decomposition of product can be suppressed, the product yield is high, the selectivity is high, and the production process has a high safety. In addition, the carboxylic acid represented by formula (2-1) can be recycled, the emission amounts of three wastes (wastewater, exhaust gas, solid waste) are low, the production costs are reduced; in particular, the product can be isolated after the reaction in a simple manner, thus the preparation method shows excellent economy. The present invention provides a method that can produce the products with a low cost while ensuring the high yield and selectivity, the method provided by the invention has the prospects for industrial applications.

According to the invention, it is preferable that R⁰ is methyl, R¹ is H or C1-C8 hydrocarbyl group.

According to the present invention, preferably, the compound represented by formula (1) is at least one selected from the group consisting of N-(1-hydroxyethyl)formamide, N-(1-hydroxyethyl)acetamide, N-(1-hydroxyethyl)propionamide, N-(1-hydroxyethyl)butanamide, N-(1-hydroxyethyl)pentanamide, N-(1-hydroxyethyl) hexanamide and N-(1-hydroxyethyl)benzamide.

According to the present invention, it is preferable that the C1-C8 hydrocarbyl group in R² is selected from the group consisting of C1-C8 alkyl group, C2-C8 alkenyl group, C2-C8 alkynyl group and benzyl group.

According to the present invention, preferably, the compound represented by formula (2-1) is at least one selected from the group consisting of formic acid, acetic acid, propionic acid, butyric acid, valeric acid, hexanoic acid, cyclohexanoic acid and benzoic acid.

According to the invention, it is preferable that the esterification reaction catalyst comprises a compound represented by formula (2-2), 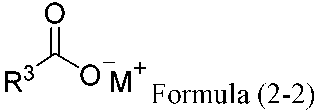
R³ in formula (2-2) is H or C1-C8 hydrocarbyl group, preferably selected from C1-C8 alkyl, C2-C8 alkenyl, C2-C8 alkynyl, benzyl or phenethyl;
M is a group IA metal element, preferably selected from Na, K or Cs.

Taking the circumstance when R⁰ is methyl as an example, the synthesis route for the preparation of N-alkenyl amide with the aforementioned method is shown below:

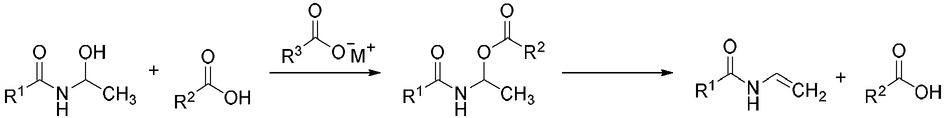

After the reaction, the carboxylic acid is not consumed and the carboxylate is used as a catalyst therein and will not be consumed. After the reaction, both the carboxylic acid and the carboxylate are recyclable, the emission amounts of three wastes (wastewater, exhaust gas, solid waste) are low, the carboxylic acid and the carboxylate can be recycled for the next reaction to prepare the N-alkenyl amide, thereby significantly reducing the production cost.

More preferably, the esterification reaction catalyst is a compound represented by the formula (2-2).

Preferably, the compound represented by formula (2-2) is at least one selected from the group consisting of sodium formate, sodium acetate, sodium propionate, sodium butyrate, sodium valerate, sodium hexanoate, sodium cyclohexanoate, sodium benzoate, potassium formate, potassium acetate, potassium propionate, potassium butyrate, potassium valerate, potassium hexanoate, potassium cyclohexanoate, potassium benzoate, cesium formate, cesium acetate, cesium propionate, cesium butyrate, cesium valerate, cesium hexanoate, cesium cyclohexanoate and cesium benzoate.

According to a particularly preferred embodiment of the present invention, R² and R³ are the same. In this way, the reaction is easier to operate, for example, by adding to a compound represented by formula (2-1) an appropriate amount of base (the metal element in the base is preferably the same metal element as that represented by M in formula (2-2) ), such that the compound represented by formula (2-1) and the compound represented by (2-2) are obtained simultaneously. Furthermore, it is ensured that the separated carboxylic acid and carboxylate after the reaction are consistent with the initially added carboxylic acid and carboxylate, it is more conducive to the recycling of carboxylic acid and carboxylate.

According to the present invention, it is preferable that the conditions of the first reaction comprise: a temperature of 8-45°C, more preferably 10-40°C (for example, it may be 10°C, 12°C, 15°C, 18°C, 20°C, 23°C, 25°C, 28°C, 30°C, 32°C, 35°C, 38°C, 40°C and a random value within the range consisting of any two numerical values mentioned above); and a time of 3-15 hours, more preferably 4-12 hours (for example, it may be 4h, 5h, 6h, 7h, 8h, 9h, 10h, 11h, 12h and a random value within the range consisting of any two numerical values mentioned above). The first reaction may be a tank reaction, which may be carried out in a flask or a reaction kettle.

Preferably, the first reaction is performed under the purging of a non-reactive gas, which is selected from nitrogen gas and/or argon gas. The non-reactive gas refers to a gas which does not participate in the reaction, and the purging of a non-reactive gas can remove water formed during the reaction process. It is also possible to blow out a small amount of acetic acid during the purging process.

According to the present invention, it is preferable that the first reaction is carried out in the presence of a water-absorbent reagent.

Preferably, the water-absorbent reagent is at least one selected from the group consisting of 4Å molecular sieve, sodium sulfate, magnesium sulfate and acetaldehyde dimethyl ether, more preferably 4Å molecular sieve.

Preferably, a mass ratio of the compound represented by formula (1) and the water-absorbent reagent in said first reaction is 1: (10-30), more preferably 1: (16-25).

After completion of the first reaction, a solid-liquid separation (e.g., filtration) may be performed to remove the water-absorbent reagent.

Preferably, the temperature of above-mentioned cracking reaction is within the range of 80-500°C. The pressure in the cracking reaction may be within the range of 0.5-1kPa (absolute pressure).

According to the present invention, it is preferred that the cracking reaction is performed in a tubular reactor. The products from the first reaction can be fed into the vessel of the cracking reaction by means of the negative pressure in order to allow the materials to pass through the cracking tube quickly.

Preferably, the temperature of the cracking reaction may be within the range of 200-350°C (e.g., 200°C, 220°C, 250°C, 270°C, 300°C, 320°C, 350°C, and a random value within the range consisting of any two numerical values mentioned above); and the time is within the range of 0.2-10 seconds (e.g., 0.2s, 0.5s, 1s, 2s, 3s, 4s, 5s, 6s, 7s, 8s, 9s, 10s, and a random value within the range consisting of any two numerical values mentioned above). The reaction tube may also be filled with fillers (e.g., alumina magnetic sphere, silicon carbide, etc.) to control the residence time of the materials in the reaction tube to obtain the above reaction time. The reactor tube may have an inner diameter of 30-50mm and a length of 400-600mm.

According to another specific embodiment, the cracking reaction may also be a tank reaction. The tank reaction may be performed at a temperature of 80-110°C for a time of 8-10 hours.

According to the present invention, the dosage molar ratio of the compound represented by formula (1), the compound represented by formula (2-1) and the esterification reaction catalyst is preferably 1: (2-25): (0.03-5), more preferably 1: (3.5-22): (0.05-0.5). Such an arrangement can further ensure the yield and selectivity of said reaction.

Wherein a part of the carboxylic acid can be removed from the solution containing the first reaction product, after the completion of the first reaction, so that the mass concentration of the first reaction product in the solution is within the range of 75-98wt%, which can further suppress coking in the cracking reaction.

It is understandable that in the conventional preparation method of a compound represented by formula (1), the obtained compound represented by formula (1) is doped with a catalyst salt due to the general need for using the catalyst salt. However, when the method provided by the invention for preparing a N-alkenyl amide using a compound represented by formula (1), the method does not require separation of the salt, it directly utilizes a compound represented by formula (1) doped with the salt, after adding the carboxylic acid, the salt is converted into the carboxylate accordingly, which can play a role of catalyst together with the additionally added carboxylate, such that a small portion of the additionally added carboxylate can be reduced. Therefore, the method provided in the present invention of using the carboxylate as a catalyst has a high tolerance for purity of the raw materials, which further simplifies the operation. The content of said catalyst doped in the compound represented by formula (1) can be determined based on the amount of catalyst added during the preparation of a compound represented by formula (1).

After the reaction is complete, the materials may be subjected to a reduced pressure distillation, the materials are initially subjected to distillation at the temperature of 30-33°C till that no liquid is evaporated (in order to separate and obtain a compound represented by formula (2), e.g. acetic acid), and then heated to a temperature of 60-70°C till that no liquid is evaporated, in order to obtain the product N-vinyl amide. The pressure of the reduced pressure distillation may be within the range of 100-200Pa.

According to the present invention, it is preferred that the method further comprises: separating N-alkenyl amide from the cracking reaction product, and recycling the remaining material for contacting with the compound represented by formula (1). As mentioned above, after removing N-alkenyl amide from the cracking reaction product, the remaining material should contain carboxylic acids and carboxylates, and possibly comprise an incompletely reacted compound represented by formula (1) and first reaction product, which can be absolutely utilized in the next process of preparing the N-alkenyl amide. The method provided by the invention that can recycle the materials, and obtain a better economic production route.

The second aspect of the present invention provides a preparation method for a N-alkenyl amide, the method comprises: under basic conditions, subjecting an aldehyde and an amide to obtain a compound represented by formula (1) through the nucleophilic addition reaction, and producing a N-alkenyl amide with the compound represented by formula (1) according to the aforementioned method; 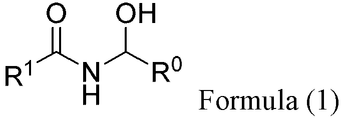 wherein R⁰ in formula (1) is C1-C5 alkyl group, R¹ is H or C1-C8 hydrocarbyl group.

It will be appreciated that specific materials and conditions involved with the first reaction and the cracking reaction have been described in the first aspect, the contents will not be repeatedly described herein.

A compound represented by formula (1) may be synthesized, for example, by reacting an aldehyde and an amide (the molar ratio of said aldehyde to said amide may be (1.2-2):1) under the alkaline conditions, for example, acetaldehyde and formamide may be reacted to obtain N-(1-hydroxyethyl)formamide, acetaldehyde and acetamide may be subjected to the reaction to produce a N-(1-hydroxyethyl)acetamide. The synthesis of said compound represented by formula (1) may be carried out in the presence of a solvent, which may be toluene, n-hexane or butanone, and the mass dosage ratio of solvent to aldehyde may be (16-25):1. The reaction may be carried out at 10-20°C for 3-6 hours, and it may be performed under the protection of nitrogen gas. The alkaline conditions may be provided by a carbonate (e.g., potassium carbonate), the molar ratio of amide to carbonate may be (50-120):1. For example, taking potassium carbonate, introducing nitrogen gas to remove oxygen gas, then adding aldehyde and a solvent, adjusting the temperature to the reaction temperature, adding (e.g., added dropwise ) the amide and controlling the temperature until the completion of said reaction. The reaction product obtained after completion of said reaction may be filtered to obtain a compound represented by formula (1).

Taking acetaldehyde and formamide as examples, the reaction route can be shown as follows:

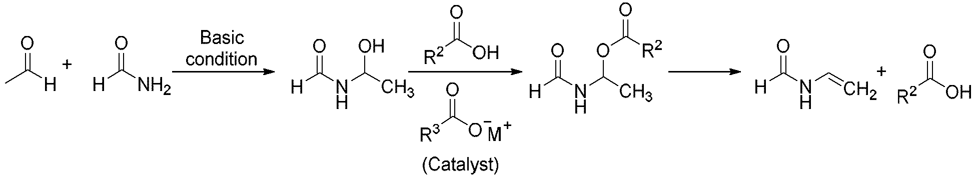

As shown in the reaction route, through the aforementioned nucleophilic addition reaction, on the one hand, a compound represented by formula (1) is obtained; on the other hand, when the alkalinity is provided by a carbonate (e.g., potassium carbonate), which can react with the raw materials R²COOH acid therein in the next step to form the carboxylate R²COOM, which is used as a catalyst for the next step of reaction. When producing a N-alkenyl amide in this way, the product obtained by the nucleophilic addition reaction does not require a complicated purification step, i.e. the process is highly tolerant to the purity of the intermediate product, and can also reduce the amount of catalyst added in the next step of reaction.

After preparing N-alkenyl amide, a polymer can also be produced using the N-alkenyl amide, the particular method can comprise: producing a N-alkenyl amide according to the aforementioned method; then using the N-alkenyl amide as monomer D' and mixing the monomer D' with monomer A', monomer B', monomer C' and an initiator to carry out the polymerization reaction under the solution polymerization reaction conditions, the polymer produced with the method has both an aqueous phase viscosifying ability and a property of emulsifying the thick oil with a low driving force, it can reduce the viscosity of thick oil and is suitable for water flooding oil reservoir exploitation. Wherein the Monomer A', monomer B', monomer C', initiator, polymerization reaction, and the like are described in detail in CN202311522670.5, CN202311522771.2, CN202311519415.5, the contents of which are specifically and entirely incorporated herein by reference.

### Preparation Example 1

Potassium carbonate (1mmol) was taken, nitrogen gas was introduced for removing oxygen gas, acetaldehyde (120mmol) and toluene (150mL) were then added, the temperature was controlled at 15°C, formamide (100mmol) was added dropwise, after the completion of the dropwise addition, the reaction temperature was kept at 15°C for carrying out the reaction for 5h; After completion, the reaction product was filtered, a (white color) solid mixture of N-(l-hydroxyethyl)formamide and potassium carbonate was prepared.

In the following examples and comparative examples:
The instrument for nuclear magnetic resonance (NMR) detection was a Bruker 500MHz NMR spectrometer.

The material obtained from the cracking reaction was subjected to reduced pressure distillation at a pressure of 100Pa, it was initially distilled at 30°C till no liquid was evaporated, acetic acid was obtained; and then distilled at 60°C till no liquid was evaporated, a product N-alkenyl amide was prepared.

In the first reaction, the first reaction product yield was calculated based on the following formula: the actual yield of the first reaction product / the theoretical production of the first reaction product ×100%, the theoretical production of the first reaction product referred to the production of the first reaction product when all the feeding amount of a compound represented by formula (1) was converted to the first reaction product. The actual yield of the first reaction product was determined by the ratio of the ¹H NMR, in particular, 50mg of the reaction liquid was added with deuterated dimethyl sulfoxide, the nuclear magnetic test was then performed. The actual yield of the first reaction product was determined based on the ratio of the peak areas of the esterification product to the peak areas of acetic acid and potassium acetate.

In the cracking reaction, conversion rate of the cracking reaction raw materials was calculated according to the following formula: consumption amount of the cracking reaction raw materials / input amount of the cracking reaction raw materials ×100%; and the cracking reaction selectivity was calculated according to the following formula: the actual yield of N-alkenyl amide / the theoretical production of N-alkenyl amide calculated based on the conversion rate of the cracking reaction raw materials.

The total yield of N-alkenyl amide was calculated according to the following formula: the first reaction product yield × the cracking reaction raw material conversion rate × cracking reaction selectivity.

### Example 1

N-(1-hydroxyethyl)formamide (100mmol, provided by a solid mixture of N-(1-hydroxyethyl)formamide obtained from the Preparation Example 1 and potassium carbonate) was taken, and placed in a 250 mL round-bottom flask, the temperature was adjusted to 10°C, acetic acid (400mmol), potassium acetate (10mmol), and 4Å molecular sieve (in a dosage of 200g) were subsequently added, after completion of adding materials, the temperature was kept at 10°C for carrying out the reaction (tank reaction) for 12h; after completion of the reaction, the molecular sieve was filtered and removed, a mixture containing N-(1-acetoxyethyl)formamide and acetic acid was obtained. The mixture was sampled, subjected to rotary evaporation to remove acetic acid, the product structure was characterized by the NMR as follows: ¹H NMR (500 MHz, DMSO) δ 8.96 (d, *J =* 7.9 Hz, 1H), 8.61 (t, *J =* 10.2 Hz, 1H), 8.21 (d, *J =* 11.0 Hz, 1H), 8.01 (s, 1H), 6.32 (dq*, J =* 8.9, 6.1 Hz, 1H), 6.01 (dq, *J =* 12.1, 6.0 Hz, 1H), 2.00 (s, 3H), 1.97 (s, 3H), it proved that the product structure was 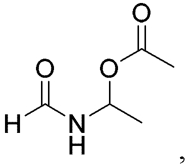 the product yield was 92.5%.

For the aforementioned mixture containing N-(1-acetoxyethyl)formamide and acetic acid, a part of the acetic acid was removed, the content of N-(1-acetoxyethyl)formamide therein was controlled to 75wt%, the mixture was used as the raw material, which was fed by means of a negative pressure, the absolute pressure was 500Pa, the cracking tube was filled with silicon carbide, the temperature inside the tube was maintained at 200°C, the reaction solution was kept in the tube for 2s (i.e., tubular reaction), after the reaction was completed, N-vinyl formamide was obtained. The cracking reaction raw material conversion rate was 95.7%, and the cracking reaction selectivity was 92.9%.

The total yield of N-vinyl formamide was 82.24%.

### Example 2

N-(1-hydroxyethyl)formamide (100mmol) was taken, and placed in a 250 mL round-bottom flask, acetic acid (1,000mmol) and potassium acetate (45mmol) were added, after completion of adding materials, the temperature was adjusted to 40°C for carrying out the reaction (tank reaction) for 4h, and the purging with nitrogen gas was performed; after completion of the reaction, a mixture containing N-(1-acetoxyethyl)formamide and acetic acid was obtained. The N-(1-acetoxyethyl)formamide yield was 90.7%.

For the aforementioned mixture containing N-(1-acetoxyethyl)formamide and acetic acid, a part of the acetic acid was removed, the content of N-(1-acetoxyethyl)formamide therein was controlled to 98wt%, the mixture was used as the raw material, which was fed by means of a negative pressure, the absolute pressure was 800Pa, the cracking tube was filled with silicon carbide, the temperature inside the tube was maintained at 250°C, the reaction solution was kept in the tube for 10s (i.e., tubular reaction), after the reaction was completed, N-vinyl formamide was obtained. The cracking reaction raw material conversion rate was 98.8%, and the cracking reaction selectivity was 90.3%.

The total yield of N-vinyl formamide was 80.92%.

### Example 3

N-(1-hydroxyethyl)formamide (100mmol) was taken, and placed in a 250 mL round-bottom flask, acetic acid (2,000mmol), potassium acetate (10mmol), and 4Å molecular sieve (in a dosage of 160g) were subsequently added, after completion of adding materials, the reaction (tank reaction) was performed at 25°C for 12h; after completion of the reaction, the molecular sieve was filtered and removed, a mixture containing N-(1-acetoxyethyl)formamide and acetic acid was obtained. The N-(1-acetoxyethyl)formamide yield was 95.2%.

For the aforementioned mixture containing N-(1-acetoxyethyl)formamide and acetic acid, a part of the acetic acid was removed, the content of N-(1-acetoxyethyl)formamide therein was controlled to 90wt%, the mixture was used as the raw material, which was fed by means of a negative pressure, the absolute pressure was 1kPa, the cracking tube lacked a filler, the temperature inside the tube was maintained at 350°C, the reaction solution was kept in the tube for 0.5s (i.e., tubular reaction), N-vinyl formamide was obtained. The cracking reaction raw material conversion rate was 99.6%, the cracking reaction selectivity was 91.7%. After the reaction was completed, N-vinyl formamide was obtained.

The total yield of N-vinyl formamide was 86.95%.

### Example 4

For the mixture containing N-(1-acetoxyethyl)formamide and acetic acid obtained in Example 1, a part of the acetic acid was removed, the content of N-(1-acetoxyethyl)formamide therein was controlled to 75wt%, the mixture was used as the raw material, which was fed by means of a negative pressure, the absolute pressure was 500Pa, the cracking tube was filled with alumina ceramic spheres, the temperature inside the tube was maintained at 390°C, the reaction solution was kept in the tube for 2s (i.e., tubular reaction), N-vinyl formamide was obtained. The cracking reaction raw material conversion rate was 98%, the cracking reaction selectivity was 77.5%.

The total yield of N-vinyl formamide was 70.25%.

### Example 5

For the mixture containing N-(1-acetoxyethyl)formamide and acetic acid obtained in Example 1, a part of the acetic acid was removed, the content of N-(1-acetoxyethyl)formamide therein was controlled to 75wt%, the mixture was used as the raw material, the reaction (tank reaction) was performed under a pressure of 1kPa and a temperature of 80°C for 9h. The cracking reaction raw material conversion rate was 96.1%, the cracking reaction selectivity was 85%.

The total yield of N-vinyl formamide was 81.7%.

### Comparative Example 1

N-(1-hydroxyethyl)formamide (100mmol) was taken, and placed in a 250 mL round-bottom flask, the temperature was adjusted to 10°C, acetic anhydride (410mmol) and 4Å molecular sieve (in a dosage of 200g) were subsequently added, after completion of adding materials, the temperature was kept at 10°C for carrying out the reaction (tank reaction) for 12h. The yield of N-(1-acetoxyethyl)formamide was 60%.

### Comparative Example 2

N-(1-hydroxyethyl)formamide (100mmol) was taken, the temperature was adjusted to 300°C for carrying out the reaction for 10h; the N-(1-hydroxyethyl)formamide conversion rate was 98.7%, the N-vinyl formamide selectivity was 2.8%, and the N-vinyl formamide yield was 2.76%.

The product structures of the products obtained from the examples were characterized by the nuclear magnetic resonance, exemplified by the product obtained in Example 1, and the data of nuclear magnetic resonance characterization ofN-vinyl formamide was as follows:

¹H NMR (500 MHz, DMSO) δ 10.13 - 9.81 (m, 2H), 8.29 (d, *J =* 10.9 Hz, 1H), 8.02 (s, 1H), 6.93 - 6.78 (m, 1H), 6.74 - 6.59 (m, 1H), 4.71 (d, *J =* 16.0 Hz, 1H), 4.52 (d, *J* = 15.5 Hz, 1H), 4.40 - 4.36 (m, 1H), 4.19 (t*, J* = 9.6 Hz, 1H).

As can be seen from the above results, an use of the method provided by the present invention have the advantages such as the operation is simple, the product yield is high, the selectivity is excellent, and the separation of products is simple, in particular, the raw materials are recyclable, thus the production costs can be greatly reduced.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A preparation method for a N-alkenyl amide, wherein the method comprises:
under an esterification reaction condition and in the presence of an esterification reaction catalyst, contacting a compound represented by formula (1) with a compound represented by formula (2-1) to carry out a first reaction, and then subjecting the obtained first reaction product to a cracking reaction; 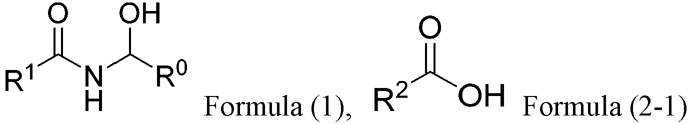
wherein R⁰ in formula (1) is C1-C5 alkyl group, R¹ is H or C1-C8 hydrocarbyl group;
R² in formula (2-1) is H or C1-C8 hydrocarbyl group.

2. The method according to claim 1, wherein R⁰ is methyl, R¹ is H or C1-C8 hydrocarbyl group;
preferably, the compound represented by formula (1) is at least one selected from N-(1-hydroxyethyl)formamide, N-(1-hydroxyethyl)acetamide, N-(1-hydroxyethyl)propionamide, N-(1-hydroxyethyl)butanamide, N-(1-hydroxyethyl)pentanamide, N-(1-hydroxyethyl) hexanamide and N-(1-hydroxyethyl)benzamide.

3. The method according to claim 1 or 2, wherein the C1-C8 hydrocarbyl group in R² is selected from C1-C8 alkyl, C2-C8 alkenyl, C2-C8 alkynyl or benzyl; preferably, the compound represented by formula (2-1) is at least one selected from formic acid, acetic acid, propionic acid, butyric acid, valeric acid, hexanoic acid, cyclohexanoic acid and benzoic acid.

4. The method according to any one of claims 1-3, wherein the esterification reaction catalyst comprises a compound represented by formula (2-2), 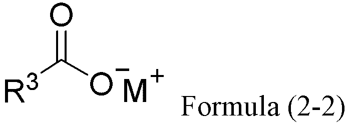
R³ in formula (2-2) is H or C1-C8 hydrocarbyl group, preferably selected from C1-C8 alkyl, C2-C8 alkenyl, C2-C8 alkynyl, benzyl or phenethyl;
M is a group IAmetal element, preferably selected from Na, K or Cs.

5. The method according to claim 4, wherein the compound represented by formula (2-2) is at least one selected from sodium formate, sodium acetate, sodium propionate, sodium butyrate, sodium valerate, sodium hexanoate, sodium cyclohexanoate, sodium benzoate, potassium formate, potassium acetate, potassium propionate, potassium butyrate, potassium valerate, potassium hexanoate, potassium cyclohexanoate, potassium benzoate, cesium formate, cesium acetate, cesium propionate, cesium butyrate, cesium valerate, cesium hexanoate, cesium cyclohexanoate and cesium benzoate.

6. The method according to claim 4, wherein R² and R³ are the same.

7. The method according to any one of claims 1-6, wherein the conditions of the first reaction comprise: a temperature of 8-45°C, preferably 10-40°C, and a time of 3-15 hours, preferably 4-12 hours.

8. The method according to any one of claims 1-7, wherein the contacting is performed under the purging of a non-reactive gas, which is selected from nitrogen gas and/or argon gas;
wherein said first reaction is carried out in the presence of a water-absorbent reagent;
preferably, the water-absorbent reagent is at least one selected from 4Å molecular sieve, sodium sulfate, magnesium sulfate and 1,1-Dimethoxyethane, more preferably 4Å molecular sieve;
preferably, a mass ratio of the compound represented by formula (1) and the water-absorbent reagent in said first reaction is 1: (10-30), more preferably 1: (16-25).

9. The method according to any one of claims 1-8, wherein the temperature of said cracking reaction is within the range of 80-500°C.

10. The method according to any one of claims 1-9, wherein the cracking reaction is performed in a tubular reactor;
preferably, the cracking reaction is performed at a temperature of 200-350°C for 0.2-10 seconds.

11. The method according to any one of claims 1-10, wherein the molar ratio of the compound represented by formula (1), the compound represented by formula (2-1) and the esterification catalyst is 1: (2-25): (0.03-5), preferably 1: (3.5-22): (0.05-0.5).

12. The method according to any one of claims 1-11, wherein the method further comprises: separating N-alkenyl amide from the cracking reaction product, and recycling the remaining material for contacting with the compound represented by formula (1).

13. A preparation method for a N-alkenyl amide, wherein the method comprises:
under basic conditions, subjecting an aldehyde and an amide to obtain a compound represented by formula (1) through the nucleophilic addition reaction, and producing a N-alkenyl amide using the compound represented by formula (1) according to the method of any one of claims 1-12; 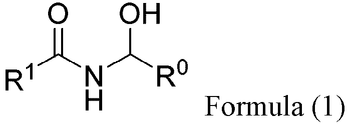
wherein R⁰ in formula (1) is C1-C5 alkyl group, R¹ is H or C1-C8 hydrocarbyl group.
